# DEMANDE DE BREVET EUROPEEN

(11) **EP 1 260 189 A2**
(43) Date de publication de la demande: **27.11.2002**
(21) Numéro de dépôt: 02291191.1
(22) Date de dépôt: 14.05.2002
(51) Int. Cl.: A61B 17/86

(54) **Vis de fixation pour réaliser le maintien d'au moins deux portions d'os l'une par rapport à l'autre**

(30) Priorité: 21.05.2001 FR 0106635
(71) Demandeur: Renard, Xavier, 91430 Vauhallan (FR)
(72) Inventeur: Renard, Xavier, 91430 Vauhallan (FR)
(74) Mandataire: Flavenot, Bernard

(57) **Abrégé**

La présente invention concerne les dispositifs de fixation permettant de solidariser entre elles temporairement au moins deux portions d'os.

Dans son application à une vis de fixation, le dispositif selon l'invention se caractérise essentiellement par le fait qu'il comporte une tige 1 dont une portion 6 comprend un filetage 2 sur au moins une partie de sa surface latérale, et un écrou 3 comportant une percée 4 suivant un axe 5, la paroi de la percée présentant un taraudage complémentaire du filetage 2 de façon que l'écrou 3 puisse se visser sur la portion de tige filetée 6, l'écrou 3 et la portion de tige filetée 6 étant réalisés respectivement dans un premier et un second matériaux ayant, à une même température donnée, des coefficients de déformation plastique différents de façon que l'un des deux matériaux, à cette température donnée, soit substantiellement plus élastique et moins malléable que l'autre.

Application, notamment, à la réalisation de vis, câbles, broches, etc. pour la réduction des fractures, par exemple de métacarpiens, de trochanters, de radius, etc.

## Description

La présente invention concerne les dispositifs de fixation pour réaliser le maintien d'au moins deux portions d'os ou deux os l'un par rapport à l'autre, comme des vis, câbles, broches ou analogues qui trouvent des applications avantageuses pour la réduction des fractures, par exemple de métacarpiens, de trochanters, de radius, etc.

On connaît déjà par exemple des vis de fixation, des câbles, des broches ou analogues permettant de maintenir deux portions d'os l'une par rapport à l'autre, par exemple les broches qui sont décrites dans les DE-U-91 05 152 et JP-A-10 052439.

Une telle broche selon l'art antérieur comporte essentiellement une tige comprenant une extrémité pénétrante et une extrémité de préhension. L'extrémité pénétrante comporte un premier filetage qui peut, ou non, être auto-taraudant. Ce premier filetage a un pas d'une valeur donnée qui permet à la tige de s'ancrer dans l'une des deux portions d'os. L'extrémité de préhension comporte, sur sa paroi latérale, un second filetage de même sens ou de sens contraire que le premier et d'un pas donné avec lequel peut coopérer un écrou qui déborde radialement de la tige, et assez largement, de façon à constituer un épaulement de surface d'appui.

Lorsque l'on veut maintenir deux portions d'os accolées l'une à l'autre, par exemple pour réduire une fracture d'un métacarpien à l'aide d'une broche telle que décrite ci-dessus, on peut commencer par réaliser, dans la première portion d'os, une percée d'un diamètre au moins égal au diamètre du premier filetage, cette percée pouvant d'ailleurs être réalisée avec l'extrémité pénétrante de la tige elle-même lorsqu'elle est auto-taraudante.

Après la réalisation de cette percée, on ancre l'extrémité pénétrante de la tige dans la seconde portion d'os de façon que l'extrémité de préhension émerge de la percée par son ouverture opposée à celle qui est la plus proche de la seconde portion d'os.

Ensuite, on visse l'écrou sur la partie de tige qui émerge de la percée jusqu'à ce que la première portion d'os soit prise en pincement entre la seconde portion d'os et l'épaulement constitué par l'écrou. En vissant plus ou moins l'écrou sur la partie de tige émergeant de la percée, on appuie les deux portions d'os plus ou moins fortement l'une contre l'autre.

Ces vis de fixation donnent de bons résultats mais peuvent présenter des inconvénients. Notamment, elles ne permettent pas d'effectuer une coupe franche et nette de l'extrémité de préhension de la tige qui dépasse de l'écrou après que la broche ait été placée, par exemple pour la réduction d'une fracture comme décrit ci-dessus. Elles ne permettent pas d'assurer assez fermement la liaison de l'écrou avec la tige en évitant qu'il se dévisse dans le temps.

Aussi, la présente invention a-t-elle pour but de réaliser un dispositif de fixation pour maintenir au moins deux portions d'os ou deux os l'un par rapport à l'autre, qui pallie au moins une grande partie des inconvénients mentionnés ci-dessus des dispositifs, dans ce domaine, de l'art antérieur.

Plus précisément, la présente invention a pour objet un dispositif de fixation permettant de maintenir temporairement au moins deux portions d'os l'une par rapport à l'autre en vue de leur solidarisation par ostéosynthèse, comportant un élément constitué d'au moins deux première et seconde pièces aptes à coopérer l'une avec l'autre pour lier les deux portions d'os l'une par rapport à l'autre, ledit élément étant apte à être coupé lorsque les deux pièces coopèrent entre elles pour lier les deux portions d'os, les deux première et seconde pièces étant réalisées respectivement dans un premier matériau et un second matériau ayant, à une même température donnée, des coefficients de déformation plastique différents de façon que l'un des deux dits matériaux, à cette température donnée, soit substantiellement plus élastique et moins malléable que l'autre.

D'autres caractéristiques et avantages de l'invention apparaîtront au cours de la description suivante donnée en regard des dessins annexés à titre illustratif mais nullement limitatif, dans lesquels :

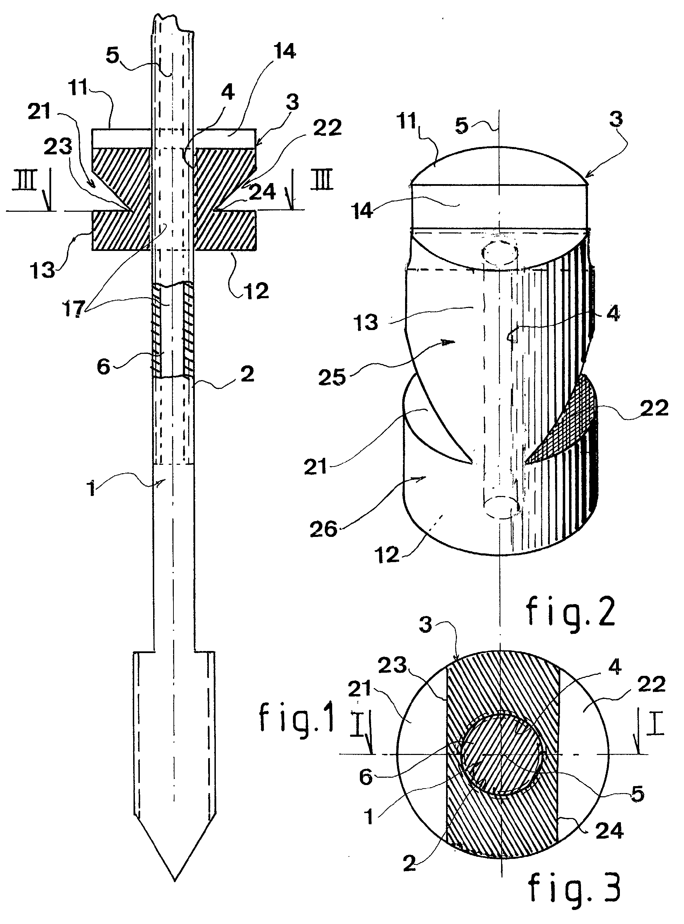

Les figures 1 à 3 représentent, sous forme schématique, un mode de réalisation d'un dispositif selon l'invention dans le cas d'une application à une vis de fixation osseuse, la figure 1 étant une vue en coupe longitudinale référencée I-I sur la figure 3 et partiellement écorchée, la figure 2 étant une vue en perspective cavalière de la partie "écrou" de la vis de fixation, et la figure 3 étant une vue en coupe transversale référencée III - III sur la figure 1, et

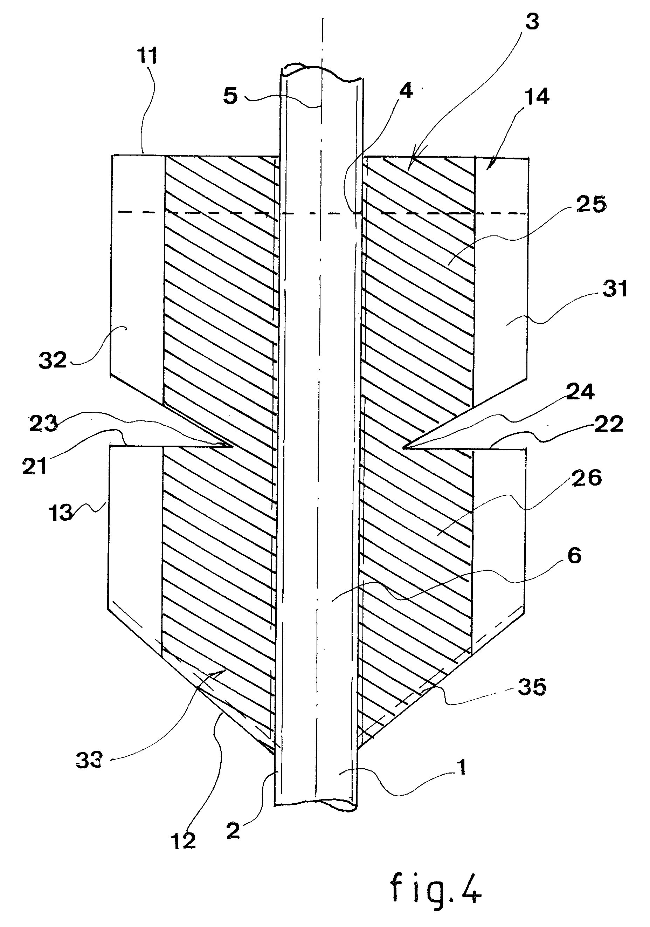

La figure 4 représente, vu en coupe longitudinale, un autre mode de réalisation du dispositif selon l'invention, dans une application à une autre vis de fixation osseuse.

Le dispositif de fixation selon l'invention permet de maintenir temporairement au moins deux portions d'os l'une par rapport à l'autre en vue de leur solidarisation par ostéosynthèse.

Ce dispositif comporte un élément constitué d'au moins deux première et seconde pièces aptes à coopérer l'une avec l'autre pour lier les deux portions d'os l'une par rapport à l'autre, l'élément étant apte à être coupé lorsque les deux pièces coopèrent entre elles pour lier les deux portions d'os, les deux première et seconde pièces étant réalisées respectivement dans un premier matériau et un second matériau ayant, à une même température donnée, des coefficients de déformation plastique différents de façon que l'un des deux matériaux, à cette température donnée, soit substantiellement plus élastique et donc moins malléable que l'autre.

Les quatre figures annexées à la présente description représentent deux modes de réalisation d'un dispositif selon l'invention dans une application respectivement à deux vis de fixation permettant de maintenir temporairement au moins deux portions d'os ou deux os l'un par rapport à l'autre en vue de leur solidarisation par ostéosynthèse, les figures 1 à 3 représentant un mode de réalisation d'une première vis de fixation et la figure 4 représentant un mode de réalisation d'une seconde vis de fixation.

Selon l'invention, dans l'application du dispositif à des vis de fixation, la première pièce est constituée par une portion 6 d'une tige 1 comprenant un filetage 2 sur au moins une partie de sa surface latérale et la seconde pièce est constituée par un écrou 3 comportant une percée 4 suivant un axe 5, la paroi de cette percée présentant un taraudage complémentaire du filetage 2 de façon que l'écrou 3 puisse se visser sur la portion de tige filetée 6.

Selon une caractéristique de l'invention, la portion de tige filetée 6 et l'écrou 3 sont réalisés respectivement dans un premier matériau et un second matériau ayant, à une même température donnée, par exemple 37° Celsius, des coefficients de déformation plastique différents de façon que l'un des deux matériaux, à cette température donnée, soit substantiellement plus élastique et moins malléable que l'autre.

De cette façon, après réduction de la fracture, il est possible de couper à la fois l'écrou 3 et la tige 1 au moyen essentiellement d'une pince coupante ou analogue, pour éliminer la partie de la vis de fixation qui émerge des portions osseuses.

En effet, comme conséquence de la caractéristique essentielle de l'invention définie ci-dessus, lors de la coupe simultanée de l'écrou et de la tige, l'un des deux matériaux va se déformer et demeurer déformé, réalisant ainsi une sorte de sertissage sur l'autre matériau. De cette façon, la tige 1 et l'écrou 3 auront moins tendance à se dévisser accidentellement tant que la fracture ne sera pas solidifiée par ostéosynthèse. En revanche, du fait de la déformation plastique uniquement de l'un des deux éléments, à savoir la tige 1 ou l'écrou 3, il sera plus facile ultérieurement de dévisser l'écrou pour ensuite enlever la tige quand la fracture sera solidifiée.

En outre, afin de favoriser le sertissage comme défini ci-dessus, il peut être avantageux que la portion de tige filetée 6 soit creuse ou analogue, comme illustré en 17 plus particulièrement sur la partie représentée en écorché de la figure 1.

Pour certains cas de fractures, il est préférable que le second matériau, celui dans lequel est réalisé l'écrou 3, soit le matériau substantiellement plus élastique et que le premier matériau, celui dans lequel est réalisée la portion de tige filetée 6, soit le matériau substantiellement plus malléable.

En revanche, pour d'autres types de fractures, il peut être préférable que le second matériau soit le matériau substantiellement plus malléable et que le premier matériau soit le matériau substantiellement plus élastique.

Dans une réalisation préférentielle, l'écrou 3 présente une forme générale sensiblement de révolution, notamment cylindrique ou tronconique, comprenant deux faces d'extrémités opposées 11, 12 et une paroi latérale 13, la percée taraudée 4 débouchant sur les deux faces d'extrémités opposées 11, 12.

L'une 11 des deux faces d'extrémités comporte au moins deux parties de rainure transversale 14 situées sensiblement dans un premier plan passant par l'axe 5 de la percée taraudée 4, de façon à pouvoir utiliser un outil du type tourne-vis à deux branches en fourche pour visser et dévisser l'écrou 3 sur la tige 1 quand celle-ci est déjà implantée.

Sur les figures 1 à 3, les deux parties de rainure sont comprises dans une seule et même rainure transversale qui, pour une facilité de réalisation, traverse en totalité la face 11. Dans cette réalisation, la profondeur de la rainure 14 est relativement faible.

Avantageusement, l'écrou comporte en outre deux rainures longitudinales 31, 32 réalisées dans sa paroi latérale 13, comme illustré sur la figure 4. Ces rainures longitudinales 31, 32 sont avantageusement situées dans le premier plan défini ci-dessus. De façon préférentielle, elles sont réalisées, toujours pour une facilité de réalisation, en continuité des parties de rainure transversale 14 et en débouchant sur les deux faces d'extrémités opposées 11 et 12.

De plus, dans le but de pouvoir aisément couper simultanément l'écrou 3 et la tige 1, il est préférable que l'écrou 3 comporte au moins une entaille 21, 22 réalisée dans sa paroi latérale 13, pour guider par exemple au moins une mâchoire de la pince coupante et mieux entamer la coupe.

Dans une réalisation avantageuse, l'entaille 21, 22 est délimitée par deux portions de plans sécants formant un dièdre, l'arête 23, 24 du dièdre étant sensiblement perpendiculaire à l'axe 5 de la percée taraudée 4.

Il est de plus préférable que l'une des deux portions de plans sécants soit perpendiculaire à l'axe 5 de la percée taraudée 4, l'autre portion de plan étant contenue dans la partie 25 de l'écrou 3 limitée par la face d'extrémité 11 comportant la rainure 14.

Pour que l'écrou conserve rigidité et solidité lors de son vissage sur la partie de tige filetée 6, il est bien entendu avantageux, comme illustré sur les figures, que l'entaille et la percée 4 n'aient pas de partie commune, afin que la percée 4 soit totalement entourée de matière.

Comme illustré sur les figures, il est préférable que l'écrou 3 comporte deux entailles 21, 22 opposées sensiblement symétriques par rapport à un second plan passant par l'axe 5 de la percée taraudée 4 et avantageusement perpendiculaire au premier plan défini ci-dessus. De cette façon, la direction de la rainure 14 permet de repérer la position des deux entailles 21 et 22 et indique au praticien comment positionner correctement la pince coupante avant d'effectuer la coupe, les deux lignes des biseaux des mâchoires de la pince devant se présenter perpendiculairement à l'axe de la rainure transversale 14.

Dans le mode de réalisation représenté sur la figure 4, lorsque l'écrou est coupé comme mentionné ci-dessus, les portions de rainures longitudinales situées dans la partie restante 26 de l'écrou limitée par la face d'extrémité 12 opposée à la face d'extrémité 11, permettent l'utilisation d'un même tourne-vis à deux branches en fourche que celui défini ci-dessus, pour dévisser cette partie restante 26 de l'écrou lorsque la fracture est consolidée.

Dans une réalisation avantageuse, la face d'extrémité 12 opposée à la face 11 peut être de forme conique 33, avec éventuellement un filet de taraudage représenté schématiquement en 35, pour favoriser une pénétration partielle de l'écrou dans l'une des parties osseuses.

L'utilisation d'une telle vis de fixation selon l'invention, communément désignée par les praticiens sous le terme de "broche" ou "fiche", ne sera pas décrite ici car elle est bien connue en elle-même et n'entre pas dans le champ de protection de l'invention.

Le dispositif peut aussi trouver une application avantageuse pour le maintien de deux portions d'os au moyen d'un cerclage par câble, technique qui est bien connue en elle-même.

En effet, il est connu que, pour maintenir ensemble deux portions d'os, par exemple suite à une fracture, on peut utiliser un câble qui entoure les deux portions d'os. Les deux extrémités du câble sont ensuite serties ensemble au moyen par exemple d'une bague ou analogue, puis les parties de câble dépassant de part et d'autre de la bague sont coupées.

De tels câbles sont constitués, pour obtenir une bonne solidité et une bonne souplesse, par au moins deux premier et second faisceaux de câbles combinés entre eux, le second entourant le premier pour former un câble unique. Cette réalisation n'a pas été spécifiquement illustrée car, comme mentionné ci-avant, elle est bien connue en elle-même.

Dans ce cas, selon l'invention, la première pièce est constituée par le premier faisceau de câbles et la seconde pièce est constituée par le second faisceau de câbles. De plus, selon l'invention, la bague est en un matériau qui est le même ou sensiblement le même que celui dans lequel est réalisé le premier faisceau de câbles, ce matériau étant plus malléable que celui dans lequel est réalisé le second faisceau.

Ainsi, quand les deux extrémités du câble unique émergeant de part et d'autre de la bague sont coupées après le sertissage de la bague, les brins du faisceau central peuvent fluer dans le second faisceau, de même que la bague, ce qui permet de parfaitement maintenir le tout comme mentionné ci-avant.

A titre d'exemple non limitatif, quelles que soient les applications du dispositif, le matériau le moins malléable peut être du TA6V qui est un alliage à base de titane, d'aluminium et de vanadium, et l'autre matériau peut être du T40 qui est du titane pur.

## Revendications

1. Dispositif de fixation permettant de maintenir temporairement au moins deux portions d'os l'une par rapport à l'autre en vue de leur solidarisation par ostéosynthèse, comportant un élément constitué d'au moins deux première et seconde pièces aptes à coopérer l'une avec l'autre pour lier les deux portions d'os l'une par rapport à l'autre, ledit élément étant apte à être coupé lorsque les deux pièces coopèrent entre elles pour lier les deux portions d'os, les deux première et seconde pièces étant réalisées respectivement dans un premier matériau et un second matériau ayant, à une même température donnée, des coefficients de déformation plastique différents de façon que l'un des deux dits matériaux, à cette température donnée, soit substantiellement plus élastique et moins malléable que l'autre.

2. Dispositif selon la revendication 1, **caractérisé par le fait que** ladite première pièce est constituée par une portion (6) d'une tige (1) comprenant un filetage (2) sur au moins une partie de sa surface latérale, et que la seconde pièce est constituée d'un écrou (3) présentant une forme générale sensiblement de révolution comprenant deux faces d'extrémités opposées (11, 12) et une paroi latérale (13), ledit écrou comportant une percée (4) suivant un axe (5), la paroi de ladite percée présentant un taraudage complémentaire dudit filetage (2) de façon que ledit écrou (3) puisse se visser sur la portion de tige filetée (6).

3. Dispositif selon la revendication 2, **caractérisé par le fait que** le second matériau, celui dans lequel est réalisé l'écrou (3), est le matériau substantiellement plus élastique et que le premier matériau, celui dans lequel est réalisée ladite portion de tige filetée (6), est le matériau substantiellement plus malléable.

4. Dispositif selon la revendication 2, **caractérisé par le fait que** le second matériau, celui dans lequel est réalisé l'écrou (3), est le matériau substantiellement plus malléable et que le premier matériau, celui dans lequel est réalisée ladite portion de tige filetée (6), est le matériau substantiellement plus élastique.

5. Dispositif selon l'une des revendications 2 à 4, **caractérisé par le fait que** ladite percée taraudée (4) débouche sur les deux faces d'extrémités opposées (11, 12), l'une (11) des deux faces d'extrémités opposées comportant au moins deux parties de rainure transversale (14) situées sensiblement dans un premier plan passant par l'axe (5) de ladite percée taraudée (4).

6. Dispositif selon la revendication 5, **caractérisée par le fait que** ledit écrou comporte en outre deux rainures longitudinales (31, 32) réalisées sur sa paroi latérale (13), lesdites deux rainures longitudinales débouchant respectivement sur les deux dites faces d'extrémités opposées (11, 12).

7. Dispositif selon l'une des revendications 2 à 6, **caractérisée par le fait que** ledit écrou (3) comporte au moins une entaille (21, 22) réalisée dans ladite paroi latérale (13), ladite entaille (21, 22) étant délimitée par deux portions de plans sécants formant un dièdre, l'arête (23, 24) dudit dièdre étant sensiblement perpendiculaire à l'axe (5) de la percée taraudée (4).

8. Dispositif selon les revendications 4 et 7, **caractérisé par le fait que** l'une des deux portions de plans est perpendiculaire à l'axe (5) de ladite percée taraudée (4), l'autre portion de plan étant contenue dans la partie (25) dudit écrou (3) limitée par la face d'extrémité (11) comportant les deux parties de rainure transversale (14).

9. Dispositif selon l'une des revendications 7 et 8, **caractérisé par le fait que** ladite entaille (21, 22) et ladite percée (4) n'ont pas de partie commune.

10. Dispositif selon la revendication 9, **caractérisée par le fait que** ledit écrou (3) comporte deux entailles (21, 22) opposées sensiblement symétriques par rapport à un second plan passant par l'axe (5) de ladite percée taraudée (4) et perpendiculaire audit premier plan.

11. Dispositif selon l'une des revendications 2 à 10, **caractérisée par le fait que** la portion de tige filetée (6) est creuse (17).

12. Dispositif selon la revendication 1, **caractérisé par le fait que** ladite première pièce est constituée par un premier faisceau de câbles et que la seconde pièce est constituée par un second faisceau de câbles, ce dit second faisceau de câbles entourant le premier pour former un câble unique de sertissage des deux portions d'os, le premier matériau étant plus malléable que le second matériau.

13. Dispositif selon la revendication 12, **caractérisé par le fait qu'**il comporte au moins une bague de sertissage apte à entourer ledit câble unique, le matériau dans lequel est réalisée ladite bague étant sensiblement identique au premier matériau.

14. Dispositif selon l'une des revendications 1 à 13, **caractérisé par le fait que** le matériau le moins malléable est du TA6V et que l'autre matériau est du T40.
